# EUROPEAN PATENT SPECIFICATION

(11) **EP 3 799 572 B1**
(45) Date of publication and mention of the grant of the patent: **26.10.2022**
(21) Application number: 18875017.8
(22) Date of filing: 26.07.2018
(51) Int. Cl.: A61M 1/00

(54) **METHOD OF FOLDING OF ASPIRATION SYSTEM LINER BAGS**
VERFAHREN ZUM FALTEN VON AUSKLEIDUNGSBEUTELN FÜR ABSAUGSYSTEME
PROCÉDÉ POUR LE PLIAGE DE SACS-DOUBLURE POUR DES SYSTÈMES D'ASPIRATION

(43) Date of publication of application: 07.04.2021
(73) Proprietor: Pergo Medikal Ve Ilaç San. A.S., Buca/Izmir (TR)
(72) Inventor: BARAN, Ozan, Buca/ zmir (TR); TAYGUR, Ahmet, Buca/ zmir (TR); YALÇIN, Ayd n, Buca/ zmir (TR); ÇOBAN, O uzhan, Buca/ zmir (TR)
(86) International application number: PCT/TR2018/050399
(87) International publication number: WO 2020/022977

(56) References cited:
- EP-A1- 1 736 183
- EP-A1- 1 984 043
- US-A- 4 516 973
- US-B2- 8 025 173

## Description

### The Related Art

Invention relates to single use liner bags in which blood and similar bodily fluids from patients are collected during operations.

The invention particularly relates to a method of folding as claimed, in which full opening of liner bag inside canister is provided following easy placement into canister before filling of said liner bags.

### Background of the Invention

Fluids such as blood or tissue fluids becoming wastes during surgery operations are collected in liner bags made of plastic material. The liner bags are kept in containers made from hard plastic called canister during their use. Canister is a container and canister is closed by liner bag put into it and unwanted fluids are discharged into the liner bag by means of connectors on the canister cap by help of tubing. The liner bag is produced to serve as a cap for the canister. In other words, after placement of the liner bag in the canister, the cap embodiment of the liner bag closes the open end of the canister. Liner bag and cap members are produced in a form in which they are integrated with each other. Vacuum is applied to the part between canister and liner bag as well as inside of the liner bag. Vacuum applied between canister and liner bag provides full opening of liner bag in the canister and sticking onto canister walls.

Said liner bags are manufactured mostly from semi-rigid plastic materials. Besides the liner bags made from semi-rigid materials, there are also liner bags made from soft materials. Disadvantage of semi-rigid materials is that it occupies great volume of place during logistics and storing. The biggest problem of the liner bags manufactured from soft materials to prevent occupation of much place during logistics and storing, is experienced during placement inside canister. Placement of liner bags kept not in order into canister causes loss of time for user. Before placement of soft liner bags into canister, the user has to shape the liner bag with hands to open it. Because the width of liner bag is the same size as the size of the canister body, it is likely to be stuck between body and cap. In addition, long size of the liner bag causes tightening in canister because of wrinkling and unbalanced folding in canister and causes failure of full seating on the canister walls. In addition to it, air pockets occur between inner wall of canister and liner bag body, which is undesirable.

Another matter which is as important for inclusion of liner bag into canister without any problem is that canister provides full opening of liner bag due to vacuum effect therein. Full contact between liner bag and canister's inner surface is not provided as a result of failure of liner bag to open fully in the prior art. In the areas having no contact, deformations in the liner bag such as bursting, tearing are seen due to vacuum effect. In other words, contact surface between two members must be uninterrupted. Full opening of liner bag in the canister and thus contact of liner bag's entire surface with inner body of canister is considerably important.

In order to eliminate the above described disadvantages, the application numbered EP07704839 and entitled "VACUUM BAG ARRANGEMENT" was filed, see EP1984043 B1. The application suggests folding the liner bag before placing the liner bag part into canister. A folded liner bag is placed into canister easily by user. However, the important point to be kept in mind for folding method is that folds are fixed by connection parts. Then connection parts are disconnected under vacuum effect within the canister. The vacuum power given here is not sometimes adequate for opening connection parts. Non-opening of connection parts means that liner bag will not be fully opened, and waste fluids will not be transferred into the liner bag.

### Purpose of the Invention

The invention is defined by the appended claims.

From the existing state of the related art, purpose of the invention is to provide quick placement of the liner bag used in aspiration systems into canister by users.

Another purpose of the invention is to eliminate the probability of non-opening of the liner bag inside the canister.

A further purpose of the invention is to provide that placement of liner bag into canister will not need user's hand talents.

Another purpose of the invention is to provide occupation of less space by said liner bag during logistic and storage stages when compared to its equivalents.

A further purpose of the invention is to provide that there is no area of no contact between liner bag and canister's internal wall together with vacuum after inclusion of liner bag into the canister.

### Brief Description of Figures

Figure 1 is side view showing fully open position of liner bag integrated with cap.
Figure 2 is side view where total length is shortened by folding liner bag inward from bottom part.
Figure 2A is side view where total length is shortened by folding liner bag inward from bottom part.
Figure 3 Is perspective view of partial folding of liner bag from mid-point of body section.
Figure 4 is side view of liner bag to be folded into two from mid-point once again after folding into two.
Figure 5 is perspective view of liner bag at stage where it is folded into four.
Figure 5A is detailed perspective view of liner bag at stage where it is folded into four.
Figure 6 Is the view of liner bag for storage with the liner bag wrapped around cap.
Figure 7 is the view of liner bag when it is folded in accordion form which is alternative folding method to wrapping around the cap.
Figure 8 is side view of liner bag before use where it is placed inside canister.

### Reference Numbers

1. Bag body
   1.1 Primary Folding line
   1.2 Secondary Folding line
2. Liner bag bottom
3. Cap
4. Connector
5. Canister

### Detailed Description of the Invention

The detailed description of the invention describes the advantageous effect of geometric changes made in liner bag body (1) of the aspiration systems liner bags, in which articles such as blood and bodily fluids from a patient during operation are collected, for placement into canister (5).

Canister (5) and liner bag body (1) cross section dimensions are close to each other. For that reason, it is not possible to place liner bags into canisters without any problems without making any geometric change on the liner bag body (1). In general application, user tries to place the liner bag into canister (5) by crushing liner bag by hand and in such case, it may cause both loss of time and inefficiency during use. Folding the geometry in a disciplined and standard way before entrance of liner bag body (1) into canister (5) provides permanency in the folded geometry and easier conduct of initial entrance of liner bag body (1) into canister (5). Patent application referred to under the title "background of the invention" is an invention made for sake of producing a solution in this subject matter.

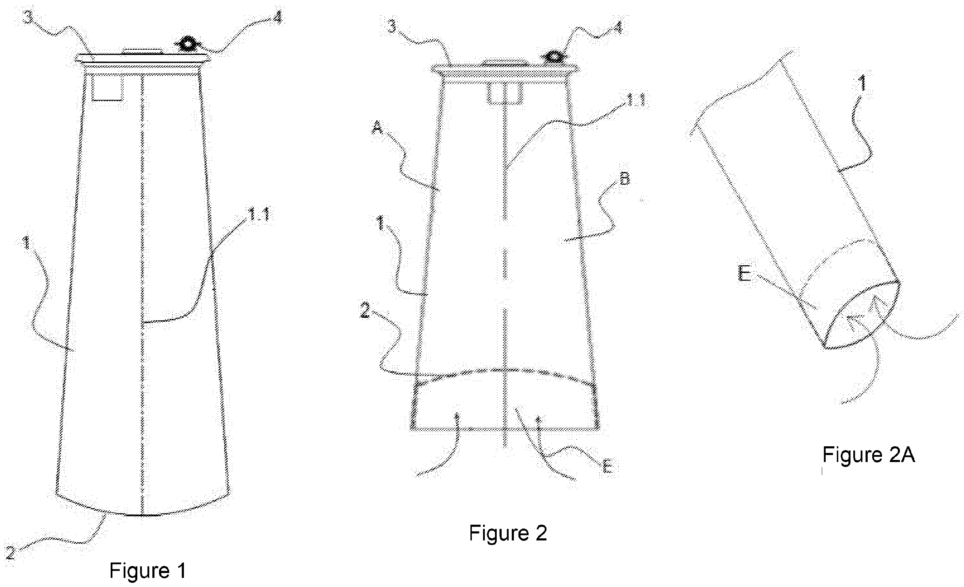

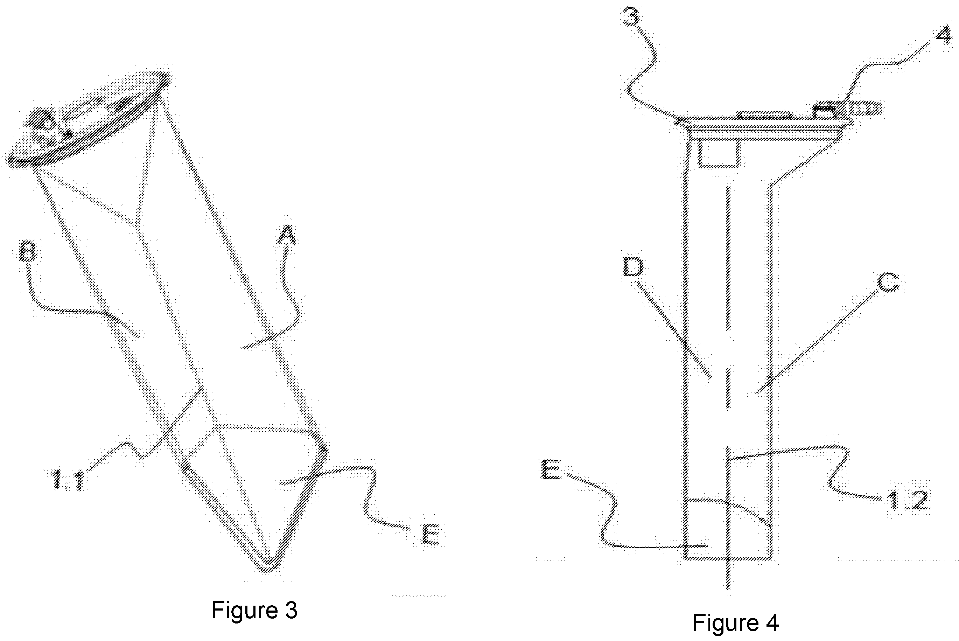

In the folding operation having a new principle, there is no apparatus on the liner bag after it enters into canister (5). As seen in figure 2, folding inwardly in arrow direction is made starting from liner bag bottom (2) part. The area formed by folding inwardly seen clearer from perspective view in Figure 3 is called Region E. Total length of the liner bag body (1) has to be longer than the canister (5) length because the liner bag should be opened and stuck onto all walls. Sticking of the liner bag body (1) onto bottom of canister (5) in cylinder form can only be provided by said length difference. The bottom surface area of cylinder to be formed when a liner bag of a certain length compressed in flat form is inflated to make a liner bag in cylinder form, is provided from side surfaces and so inflated cylinder length gets shortened. An initially flat liner bag of same length as cylindrical canister (5), when inflated will remain far from the bottom of canister (5) in the amount of said length shortening and will have a volume smaller than specified volume of canister (5).

The necessary length causes irregular blocking in the canister (5) and failure of liner bag to open to bottom fully. Folding of the liner bag body (1) inwardly provides location of liner bag required to be long inside the canister (5) having a standard geometry and proper opening by help of vacuum applied between the canister (5) and the liner bag body (1).

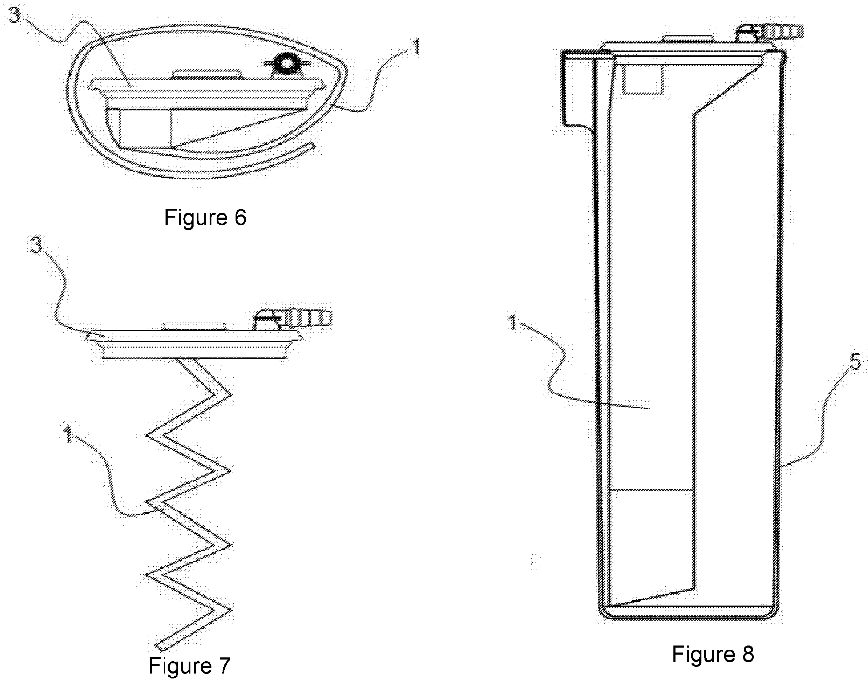

After folding the liner bag body (1) inwardly, the liner bag body (1) is folded from the middle starting from liner bag bottom (2) and a primary folding line progressing towards cap (3) is formed. The parts on the right and left of the primary folding line (1.1) are called regions A and B. Surfaces A and B are folded at primary folding line (1.1) center in a manner they overlap as seen in figure 3. After said folding the liner bag body (1) takes the form as shown in Figure 4, liner bag body (1) brought into this geometry is easily included into the canister (5) as shown in figure 8. As this primary folding line (1.1) approaches cap (3) section, it loses its effect, but this case is not very important. Said effect is shown clearly in figure 4. Liner bag body (1) part is reduced into exactly half of the broadest width. After liner bag body (1) part is folded into two, following its easy entrance into canister (5), liner bag body (1) together with vacuum contacts wall of canister (5) and fills in the inner volume of the canister (5) without any problems. Since liner bag body (1) has no connection member and folding geometry is composed of one single operation, no problem is experienced in fully opening of the liner bag body (1) after vacuum is applied between liner bag and canister (5). Then fluids from the connector (4) can be transferred into liner bag body (1) easily.

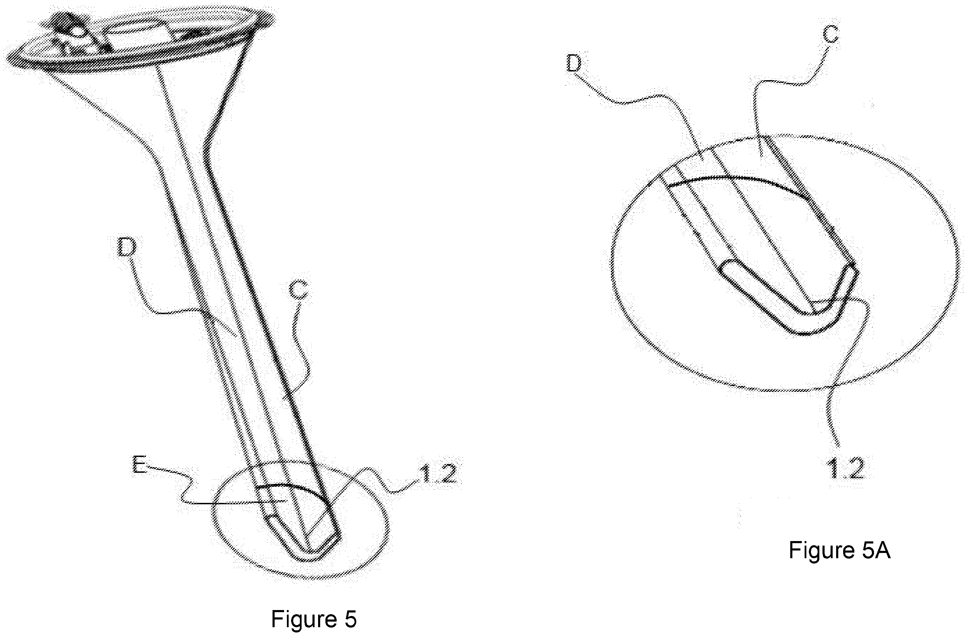

In order to make the easy operation provided by means of folding the liner bag body (1) into two around the primary folding line (1.1) as described in the above paragraph more problem free, a secondary folding line (1.2) is provided at mid point of Areas A and B on the folded in the position on top of each and total area covered by the liner body (1) is reduced by half once again. In other words, total area reduced by half by folding line (1.1) is reduced by half once again by second folding line (1.2). Thus, Areas A and B are folded into two, namely areas C and D again. Figure 5 shows second folding operation and areas C, D formed thereby. After overlapping of areas A and B, it is folded around a second folding line (1.2) and areas C and D are formed as shown in details in Figure 5A. Areas C and D are folded in a manner they overlap each other in the final step, and folding liner bag body (1) into four is provided and entrance into canister (5) may become much easier.

When stocking for storage purpose is required, open form of liner bag body (1) shows negative effects. For stocking, requirement is to occupy as little space as possible. To provide it, after the liner bag body (1) is folded into two or four along folding line (1.1) as described above, liner bag body (1) is wrapped around cap (3) as shown in figure 6. Thus only about cap (3) size area is occupied. After wrapping the liner bag body (1) around the cap (3), it is fixed by a flexible object such as rubber band. In order to use the liner bag, the user firstly needs to remove the rubber band or similar object. Then although the liner bag body (1) is released from the cap (3), it will not lose its form folded into two and will take the position shown in figure 5. Placement of liner bag body (1) of this position into the canister (5) is very easy.

An alternative stacking position is shown in figure 7. In this method, after the liner bag body (1) is folded from primary folding line (1.1), it is folded in accordion form and connected to cap (3) section. Connection can be made by use of a rubber band. After removal of rubber band before use, the liner bag body (1) comes into a position as shown in figure 5 and can be placed into canister easily.

## Claims

1. A folding method for liner bags that are produced integral with a cap (3) and which are used as a disposable in aspiration systems where fluids are collected from a body during surgery , said liner bags being made of plastic material, which perform their functions while inside a canister (5), the method being **characterized in that** it consists of the following method steps:
• folding from the liner bag bottom (2) part towards an internal part of the liner bag body (1) and
• folding into two from mid-point starting from the liner bag bottom (2) part of the liner bag body (1) and folding in the form of a straight line to the cap (3) part around a primary line (1.1) formed on the liner bag body (1).

2. A folding method for liner bags according to claim 1 **characterized in that** it facilitates the operation of the entrance of said liner bag body (1) into said canister (5):
• by folding the bag body (1) into two from the first folding line (1.1)
• and by folding the liner bag body (1) into totally four along a second folding line (1.2).

3. A folding method for liner bags according to claim 1 **characterized in** tightly wrapping the liner bag body (1) around the cap (3) in order to ensure that said liner bag body (1) occupies less space when outside the canister (5).

4. A folding method for liner bags according to claim 1 **characterized in that** said liner bag body (1) is folded in accordion shape and collected and fixed under said cap (3) in order to provide occupation of less space when said liner bag body (1) is outside said canister (5).

## Patentansprüche

1. Faltverfahren für Auskleidungsbeutel, welche einstückig mit einer Kappe (3) hergestellt werden und welche als Einweg in Absaugsystemen verwendet werden, in denen Flüssigkeiten während einer Operation aus einem Körper gesammelt werden, und die genannten Auskleidungsbeutel aus Kunststoffmaterial hergestellt sind, die ihre Funktionen erfüllen, während sie sich in einem Kanister (5) befinden, wobei das Verfahren **dadurch gekennzeichnet ist, dass** es aus folgenden Verfahrensschritten besteht:
• Faltung vom Auskleidungsbeutelboden (2) zu einem Innenbereich des Auskleidungsbeutelkörpers (1) und
• Faltung in zwei Hälften von der Mitte aus, ausgehend von dem Teil des Auskleidungsbeutelbodens (2) des Auskleidungsbeutelkörpers (1) und Faltung in Form einer geraden Linie zum Teil der Kappe (3) um eine auf dem Auskleidungsbeutelkörper (1) gebildete Hauptlinie (1.1)

2. Faltverfahren für Auskleidungsbeutel nach Anspruch 1, **dadurch gekennzeichnet, dass** es den Vorgang des Einführens des Auskleidungsbeutelkörpers (1) in den Kanister (5) erleichtert:
• durch Falten des Beutelkörpers (1) in zwei Teile an der ersten Faltlinie (1.1),
• und durch Falten des Auskleidungsbeutelkörpers (1) in insgesamt vier Teile entlang einer zweiten Faltlinie (1.2).

3. Faltverfahren für Auskleidungsbeutel nach Anspruch 1, **dadurch gekennzeichnet, dass** der Auskleidungsbeutelkörper (1) eng um die Kappe (3) gewickelt wird, um sicherzustellen, dass der Auskleidungsbeutelkörper (1) außerhalb des Kanisters (5) weniger Raum einnimmt.

4. Faltverfahren für Auskleidungsbeutel nach Anspruch 1, **dadurch gekennzeichnet, dass** der Auskleidungsbeutelkörper (1) in Ziehharmonikaform gefaltet und unter der Kappe (3) gesammelt und befestigt wird, um einen geringeren Platzbedarf zu gewährleisten, wenn sich der Auskleidungsbeutelkörper (1) außerhalb des Kanisters (5) befindet.

## Revendications

1. Procédé de pliage pour les sacs intérieurs qui sont produits solidaires d'un capuchon (3) et qui sont utilisés comme jetable dans les systèmes d'aspiration où des fluides sont collectés à partir d'un corps lors d'une intervention chirurgicale, lesdits sacs intérieurs étant réalisées en matière plastique, qui remplissent leurs fonctions à l'intérieur d'un réservoir (5), le procédé étant **caractérisé en ce qu'**il se compose des étapes de procédé suivantes :
• pliage de la partie inférieure du sac intérieur (2) vers une partie interne du corps du sac intérieur (1) et
• pliage en deux à partir du point médian en partant de la partie inférieure (2) du sac intérieur du corps du sac intérieur (1) et pliage sous la forme d'une ligne droite jusqu'à la partie capuchon (3) autour d'une ligne primaire (1.1) formée sur le corps du sac intérieur (1).

2. Procédé de pliage de sacs intérieurs selon la revendication 1, **caractérisé en ce qu'**il facilite l'opération d'entrée dudit corps de sac intérieur (1) dans ledit réservoir (5):
• en pliant le corps de sac (1) en deux à partir de la première ligne de pliage (1.1),
• et en pliant le corps de sac intérieur (1) en quatre au total le long d'une deuxième ligne de pliage (1.2).

3. Procédé de pliage de sacs intérieurs selon la revendication 1, **caractérisé par** l'enroulement serré du corps de sac intérieur (1) autour du capuchon (3) afin de s'assurer que ledit corps de sac intérieur (1) occupe moins d'espace lorsqu'il est à l'extérieur du réservoir (5).

4. Procédé de pliage de sacs intérieurs selon la revendication 1, **caractérisé en ce que** ledit corps de sac intérieur (1) est plié en forme d'accordéon et collecté et fixé sous ledit capuchon (3) pour occuper moins d'espace lorsque ledit corps de sac intérieur (1) est à l'extérieur dudit réservoir (5).
